# EUROPEAN PATENT SPECIFICATION

(11) **EP 2 370 023 B1**
(45) Date of publication and mention of the grant of the patent: **05.09.2012**
(21) Application number: 09760592.7
(22) Date of filing: 21.10.2009
(51) Int. Cl.: A61F 2/08

(54) **COLLAGEN-BASED TENDON REPLACEMENT IMPLANT**
SEHNENERSATZIMPLANTAT AUF KOLLAGENBASIS
IMPLANT À BASE DE COLLAGÈNE POUR LE REMPLACEMENT DE TENDONS

(30) Priority: 22.10.2008 FR 0805852
(43) Date of publication of application: 05.10.2011
(73) Proprietor: Sofradim Production, 01600 Trevoux (FR)
(72) Inventor: BAYON, Yves, F-69007 Lyon (FR); GRAVAGNA, Philippe, F-69540 Irigny (FR); MENEGHIN, Alfredo, F-69009 Lyon (FR)
(74) Representative: Maureau, Philippe
(86) International application number: PCT/IB2009/007527
(87) International publication number: WO 2010/046787

(56) References cited:
- WO-A-92/03988
- WO-A-95/01810
- FR-A- 2 651 994
- FR-A- 2 846 549

## Description

The present invention relates to an implant intended to reinforce or replace a defective orthopaedic soft tissue having, for example, a role of connecting between a bone and a muscle or between two bones of the human body, such as for example tendons or else ligaments.

Such tissues, such as for example the rotator cuff of the shoulder joint, are regularly stressed during everyday movements and they must have good mechanical strength properties.

However, when these tissues are overstressed or when they have been victims of a trauma, they can tear and it is then necessary to repair them. However sometimes the tears in tendons or ligaments are too large for the two separated parts of the tendon or of the ligament to be able to be joined back together using sutures or staples. In such a case, it is necessary to use grafts or implants to make up for the lack of tissue between the two parts separated by the tear.

Autografts or allografts are commonly used in so far as they have the advantage of offering the required mechanical properties and that they are similar, anatomically speaking, to the tissues to be repaired.

However, most of the time, the grafts do not satisfactorily withstand the growth of the newly formed tendons and/or ligaments. They cannot be significantly remodelled due to their properties that are inherent to their nature, especially due to their low porosity, preventing an early infiltration of the cells and an adequate vascularization of the graft, this early infiltration and this vascularization nevertheless being necessary for the regeneration of the defective tendons and ligaments.

Biosynthetic implants have been developed and proposed for the purpose of overcoming the drawbacks linked to grafts. Such implants are, for example, in the form of biocompatible textiles implanted at the fault in the defective tissue. Thus, document US 2003/0225355 describes an implant based on a bioresorbable collagen matrix which may confine a textile. Document EP 1 216 718 describes an implant comprising a bioresorbable polymeric sponge reinforced by a textile. Document US 6,262,332 describes a biomaterial comprising a layer of non-human collagen and a textile. However, these implants, whether they are resorbable or not, do not always allow satisfactory cell growth: in particular, they do not allow a homogeneous, controlled and gradual cell colonization.

Document FR 2 846 549 A1 describes a porous textile coated by a bioresorbable material. Document WO95/01810 describes a bioresorbable matrix material seeded with fibroblasts.

There is therefore the need for an implant for reinforcing or replacing orthopaedic soft tissues, such as tendons or ligaments, having at the same time mechanical properties, and in particular tensile strength and breaking strength, that are sufficient so that said implant can carry out its very stressed connective role between two components of the body and at the same time a porosity that allows and that favours a cell colonization that is simultaneously homogeneous, controlled and gradual.

In particular the need remains for such an implant which would limit the long-term presence of a foreign body in the organism, while allowing such a controlled and gradual cell colonization so that the regeneration of the defective tissue takes place during the time that the implant is still significantly present at the defect to be filled in.

The present invention relates to an implant intended to replace or reinforce defective orthopaedic soft tissue, such as a tendon or ligament, comprising:
- a bioresorbable porous matrix based on a collagen sponge;
- a porous three-dimensional knit having a longitudinal direction, characterized in that:
- said porous matrix fills said three-dimensional knit, said three-dimensional knit having two opposite faces, a front face and a rear face, connected to one another by a spacer, at least one of said front and rear faces has at least one weave of pillar stitch type in the longitudinal direction of said knit.

The fact that the porous matrix contains the knit, which is also porous, makes it possible to give the implant an overall porosity that enables optimal cell colonization. Furthermore, the implant according to the invention has a tensile strength in its longitudinal direction such that it can perfectly carry out its role of reliable connective component, for example between a bone and a muscle or between two bones at a joint, such as the shoulder joint. In particular, in said longitudinal direction, a knit has an almost zero elasticity. Thus, when the implant is stressed in this direction, it responds immediately and the movements demanded at the joint are carried out correctly and reliably.

According to the present application, the term "implant" is understood to mean a biocompatible medical device that can be implanted in the human or animal body.

According to the present application, the expression "orthopaedic soft tissues" encompasses tendons and ligaments.

The implant according to the invention comprises a bioresorbable porous matrix based on a collagen sponge.

According to the present application, the term "bioresorbable" is understood to mean the characteristic according to which a material is bioresorbed or degraded by the surrounding biological tissues and fluids and disappears *in vivo* after a given period of time which may vary, for example, from one day to several months, as a function of various factors such as the chemical nature of the material, its degree of crosslinking, its physical characteristics in the implant such as its dimensions (e.g. its thickness), its density and its porosity.

In the present application, the term "porous" is understood to mean the characteristic according to which a structure has pores, holes, cavities or voids, which are open, which may be distributed evenly or unevenly, and which promote all cell colonization. In the case of a knit, as will be seen later on, these pores, holes, cavities or voids may constitute channels that emerge on both sides of the knit.

According to the present application, the term "sponge" is understood to mean a porous structure comprising pores, like a foam, obtained in particular by freeze-drying a solution or a suspension of one or more compounds. By "based on a collagen sponge" is meant according to the present application that the bioresorbable porous matrix of the implant of the invention includes at least a collagen sponge.

According to the present application, the term "collagen" is understood to mean any known collagen of porcine, bovine or human origin, for example natural collagen, esterified collagen, for example methylated, ethylated or else succinylated collagen, or one of its derivatives, which may or may not be heated, which may or may not be oxidized or else which may be crosslinked with another compound.

According to the present application, the expression "natural collagen" is understood to mean a collagen which has not been chemically modified, other than by a possible treatment with pepsin in order to digest the telomeric peptides.

The collagen sponge of the present invention is preferably obtained by freeze-drying a solution of collagen or of a mixture of collagens.

The implant according to the invention also comprises a porous three-dimensional knit having a longitudinal direction.

According to the present application, the expression "three-dimensional knit" is understood to mean an assembly or an arrangement of biocompatible monofilament or multifilament yarns, obtained by knitting and that has a significant thickness, preferably greater than or equal to 0.5 mm.

The expression "longitudinal direction" is understood according to the present application to mean the direction that coincides with the largest dimension of the three dimensions of the three-dimensional knit.

In the implant according to the invention, the matrix fills the three-dimensional knit, that is to say that the three-dimensional knit is completely contained within the porous matrix. The three-dimensional knit of the implant according to the invention has preferably a knit weave capable of giving it an elongation at 50 N in said longitudinal direction that is strictly less than 10%, measured according to the ISO 13934-1 Standard. In one embodiment of the invention, this elongation ranges from 0.5 to 8%. The three-dimensional knit of the implant according to the invention preferably has an elongation at 100 N in said longitudinal direction that is strictly less than 20%, measured according to the ISO 13934-1 Standard. In one embodiment of the invention, this elongation at 100 N ranges from 1 to 15%.

The particular configuration of the implant according to the invention, namely the combination of a porous matrix based on a collagen sponge and of a porous three-dimensional knit trapped within this matrix and having, furthermore, a weave of pillar stitch type on one of its faces that gives it a particular elongation in its longitudinal direction, enables the implants to promote, on the one hand, a homogeneous, gradual and controlled cell growth and, on the other hand, to provide an excellent mechanical strength in its longitudinal direction: thus, as certain parts of the implant, such as for example the bioresorbable porous matrix, degrade, the cell regeneration can gradually develop at the sites left vacant by this partial bioresorption in order to, little by little, replace the resorbed parts of the implant, the implant furthermore continuing simultaneously to play its reinforcing role due to the parts of the implant not yet bioresorbed or that are intended to remain permanently within the body of the patient. Thus, preferably, in the case of the replacement of a tendon or of a ligament, the implant according to the invention will be positioned so that the longitudinal direction of the implant coincides with the strain direction of the tendon or of the ligament, that is to say the direction along which the tendon or the ligament is stressed.

Pillar stitch weaves are well known to a person skilled in the art. Such a pillar stitch weave, present in the longitudinal direction on one of the faces of the three-dimensional knit of the implant according to the invention, makes it possible to give the knit very good tensile strength in its longitudinal direction. Thus, the implant according to the invention has a low elasticity in its longitudinal direction. Thus, when the implant is stressed in its longitudinal direction, it responds immediately and the joint within which the implant is implanted can correctly play its role.

The three-dimensional knit of the implant of the invention comprises two opposite faces, a front face and a rear face, connected to one another by a spacer, at least one of said front and rear faces having at least one weave of pillar stitch type. In the present application, the term "spacer" is understood to mean the lap or laps of yarns which connect(s) the two faces of the knit to one another, thus constituting the thickness of the knit.

In one embodiment of the invention, each of the front and rear faces of said three-dimensional knit has at least one pillar stitch type weave. Such a configuration makes it possible to obtain a three-dimensional knit that is particularly not very elastic in its longitudinal direction.

One embodiment of the invention, the collagen is in the form of a mixture of at least one collagen which undergoes rapid in vivo bioresorption and of at least one collagen which undergoes slow in vivo bioresorption.

The expression "collagen which undergoes slow *in vivo* bioresorption or biodegradation" is understood to mean a collagen capable of being completely bioresorbed or degraded *in vivo,* that is to say within the human body, over unacceptable and controllable duration ranging from around 3 months to 12 months. The expression "collagen which undergoes rapid *in vivo* bioresorption or biodegradation" is understood to mean a collagen capable of being completely bioresorbed or degraded *in vivo,* that is to say within the human body, over an adaptable and controllable duration ranging from around 1 day to 3 months, preferably from 1 week to 8 weeks.

When the collagen used is crosslinked, for example by a crosslinking agent, the degree of crosslinking of the collagen will have an effect on the *in vivo* resorption rate of the crosslinked collagen. Consequently, a crosslinked collagen may be used either as a "collagen which undergoes slow *in vivo* bioresorption or biodegradation" or as a "collagen which undergoes rapid *in vivo* bioresorption or biodegradation" depending on its degree of crosslinking. In particular, the more a collagen is crosslinked, that is to say the higher its degree of crosslinking, the slower its *in vivo* bioresorption.

In one embodiment of the invention, the collagen which undergoes slow *in vivo* bioresorption is a collagen crosslinked with a crosslinking agent chosen from glutaraldehyde, hexamethylene diisocyanate (HMDI), bifunctional or trifunctional glycidyl ethers, carbodiimides, acyl azides, divinyl sulphone and mixtures thereof, the degree of crosslinking of said crosslinked collagen giving it a bioresorption time ranging from 3 months to 12 months. For example, said collagen which undergoes slow *in vivo* bioresorption is a collagen crosslinked with glutaraldehyde. In another embodiment of the invention, said collagen which undergoes slow *in vivo* bioresorption is a collagen crosslinked with hexamethylene diisocyanate (HMDI).

In one embodiment of the invention, the collagen which undergoes rapid *in vivo* bioresorption is an oxidized collagen or a collagen crosslinked with a crosslinking agent chosen from glutaraldehyde, bifunctional or trifunctional glycidyl ethers, carbodiimides, acyl azides, divinyl sulphone and mixtures thereof, the degree of crosslinking of said crosslinked collagen giving it a bioresorption time ranging from 1 day to 3 months, for example ranging from 1 to 8 weeks. For example, the collagen which undergoes rapid *in vivo* bioresorption is an oxidized collagen.

In one such embodiment of the invention, in which the collagen is in the form of a mixture of at least one collagen which undergoes rapid bioresorption and of at least one collagen which undergoes slow bioresorption, the collagen sponge matrix has, once implanted, two-speed resorption kinetics, with one part of its structure which is resorbed more quickly than the other part. Such an embodiment thus makes it possible to create, in a gradual and controlled manner, new pores, interconnected with the pores that already exist, that the cells will gradually colonize as the part made of collagen which undergoes rapid bioresorption degrades. The cell growth will thus be carried out gradually and homogeneously. Such an embodiment also makes it possible to increase the interconnectivity of the implant over time and thus to improve the tissue integration of the implant.

According to the present application, the expression "interconnected pores" is understood to mean open pores which are connected to one another and communicate with one another in the whole of the implant, without separation, so that a cell that is in one pore can pass from one pore to the other, in the whole of the implant, and can in theory circulate through all the pores in the entire implant.

The term "interconnectivity" is understood in the sense of the present application to mean the ability of the implant to allow any cell that is in a pore to circulate within all the other pores of the implant. Thus, in the case of complete interconnectivity, all the pores of the implant are accessible to any cell originating from the organism in which the implant is implanted.

In one embodiment of the invention, said matrix defining first pores, and said knit defining second pores, said first and second pores are at least partially interconnected with one another.

According to the present application, the expression "pores at least partially interconnected" is understood to mean that certain pores, for example from 0.1 to 80% of all of the pores, may be closed and do not communicate with the adjacent pores. The open or closed nature of the pores may be evaluated, for example by electron microscopy.

In its initial state, before implantation, the implant according to the invention may be such that all of its pores, that is to say the first and the second pores are all completely interconnected. In another embodiment of the invention, the implant may be such that in its initial state, before implantation, all of its pores, that is to say the first and the second pores are partially interconnected, that is to say that certain pores are closed to communication with the adjacent pores. In such a case, the gradual degradation *in vivo* of the various elements constituting the implant, and in particular of the collagen sponge, allows the pores that were initially closed to be opened little by little. After sufficient partial degradation *in vivo* after implantation, all the pores, the first and the second pores, become completely interconnected.

In one embodiment of the invention, said knit comprises at least one non-resorbable material and at least one bioresorbable material.

According to the present application, the expression "non-bioresorbable" is understood to mean the feature according to which an implant or a material retains its initial mechanical properties after a period of 2 years during which it is placed in contact with biological tissues, for example within a human organism.

For example, the knit comprises at least 50%, by weight, relative to the total weight of the knit, of bioresorbable material.

In such an embodiment, the bioresorption of the bioresorbable part of the knit also participates in the gradual degradation of the bioresorbable parts of the implant according to the invention and it helps with the control and homogeneity of the cell colonization. Furthermore, whereas the collagen forming the sponge of the matrix and the bioresorbable part of the knit degrade *in vivo* gradually, for example one after the other, the implant retains a significant portion of its mechanical strength owing to the non-bioresorbable part of the knit which does not degrade.

In another embodiment of the invention, said knit is composed of bioresorbable material, that is to say is completely bioresorbable. For example, the knit may then comprise at least one bioresorbable material with slow *in vivo* bioresorption, so that the implant retains its mechanical strength due to the material with slow bioresorption which degrades more slowly than the collagen or the mixture of collagen forming the sponge of the matrix of the implant according to the invention. When the implant is completely bioresorbed, the organic tissue newly formed during the cell regeneration is sufficient to take over from the implant and provide the function of connective element between two organs of the human body.

In one embodiment of the invention, the implant also comprises a bioresorbable film on at least one of its faces. Such a film preferably has a smooth anti-adhesive surface and is particularly suitable for the manufacture of an implant for reinforcing or replacing orthopaedic soft tissues, also having anti-adhesive properties. For example, the implant according to the invention may comprise a bioresorbable film on each of its two faces, so that said matrix is sandwiched between the two bioresorbable films.

The invention will be better understood from the description which follows, with reference to the appended drawing:
Figures 1 a and 1 b respectively represent knit weaves for the front (Figure 1a) and rear (Figure 1 b) faces of a three-dimensional knit of an implant according to the invention;
Figure 2 represents the knit weave of the spacer of a three-dimensional knit of an implant according to the invention; and
Figures 3a and 3b respectively represent the knit weaves for the front (Figure 3a) and rear (Figure 3b) faces of another embodiment of a three-dimensional knit of an implant according to the invention.

The implant according to the invention comprises a bioresorbable porous matrix based on a collagen sponge that defines first pores. Such a sponge is preferably obtained by freeze-drying a suspension of collagen. The sponge obtained has pores, or voids, cavities, holes or orifices which may or may not be evenly distributed, and which are more or less interconnected, depending on the freeze-drying process used. Such freeze-drying processes are known. It is known to vary the temperature and the rate of freezing and also the characteristics of the collagen solution or suspension to be freeze-dried (pH, concentration, etc.) as a function of the structure of the sponge that it is desired to obtain (see US 4970298 ; Doillon et a/., J Biomed Mater Res, 1986 ; Schoof, J Biomed Mater Res, 2001; O'Brien et al., Biomaterials, 2004).

Preferably, in the implant according to the invention, the first pores, defined by said sponge, are homogeneously distributed within the matrix. These first pores may, for example, have an average diameter ranging from 50 to 500 µm. These first pores, defined within the sponge on its own, in the absence of the knit of the implant according to the invention, may or may not be interconnected with one another.

The collagen sponge of the matrix of the implant according to the invention is preferably obtained from a mixture of at least one collagen which undergoes slow *in vivo* bioresorption and at least one collagen which undergoes rapid *in vivo* bioresorption.

The collagen which undergoes slow *in vivo* bioresorption may be chosen from any collagen, which is pure or derived, which may or may not be heated, and which may or may not be oxidized, having a bioresorption or biodegradation time of between 3 and 12 months. For example, the collagen which undergoes slow *in vivo* bioresorption may be a collagen crosslinked with a crosslinking agent chosen from glutaraldehyde, hexamethylene diisocyanate (HMDI), bifunctional or trifunctional glycidyl ethers, carbodiimides, acyl azides, divinyl sulphone and mixtures thereof, the degree of crosslinking of said crosslinked collagen giving it a bioresorption time ranging from 3 months to 12 months. It may also be obtained by crosslinking of the collagen by physical methods such as photooxidation. The crosslinking of the collagen by various crosslinking agents has been the subject of numerous studies and a person skilled in the art knows what degree of crosslinking to attribute to a collagen so that the latter has a slow bioresorption according to the present application, that is to say ranging from 3 to 12 months.

In one embodiment of the invention, use is made, as collagen which undergoes slow *in vivo* bioresorption, of collagen crosslinked with glutaraldehyde. Such a collagen, having a degree of crosslinking determined by a person skilled in the art that is sufficiently high for a slow bioresorption, may for example be obtained by incubation of a solution of collagen neutralized with a solution of glutaraldehyde, removal of excess glutaraldehyde and neutralization in order to obtain a precipitate of collagen crosslinked with glutaraldehyde.

In another embodiment of the invention, use is made, as collagen which undergoes slow *in vivo* bioresorption, of collagen crosslinked with hexamethylene diisocyanate (HMDI). Such a collagen, having a degree of crosslinking determined by a person skilled in the art that is sufficiently high for a slow bioresorption, may for example be obtained by incubation of a suspension of collagen in a solvent (for example dimethyl sulphoxide (DMSO), isopropanol, propanol or acetone), with a solution of HMDI, removal of excess HMDI and of the by-products of HMDI, in order to obtain dry fibres of collagen crosslinked with HMDI. For example, a collagen crosslinked with HMDI having the correct degree of crosslinking for a slow bioresorption according to the definition of the present application may be obtained in the following manner: 50 g of dry porcine collagen is mixed with 1 l of acetone. 1 g of HMDI is then added to the suspension of collagen. The mixture is left overnight, with stirring and at ambient temperature (around 20°C). The collagen fibres are then recovered by filtering the suspension through a nylon mesh and are washed carefully with dry acetone to remove the HMDI and the by-products of HMDI which are soluble in acetone. The crosslinked collagen fibres thus obtained are dried by removing the residues of acetone. The fibres may then be optionally ground.

The collagen which undergoes rapid *in vivo* bioresorption may be chosen from any collagen, which is pure or derived, which may or may not be heated, and which may or may not be oxidized, having a bioresorption or biodegradation time of between 1 day and 3 months, preferably between 1 day and 8 days. For example, the collagen which undergoes rapid *in vivo* bioresorption may be an oxidized collagen or a collagen crosslinked with a crosslinking agent chosen from glutaraldehyde, bifunctional or trifunctional glycidyl ethers, carbodiimides, acyl azides, divinyl sulphone, collagen crosslinked by UV, beta or gamma irradiation or by heat treatment and mixtures thereof, the degree of crosslinking of said crosslinked collagen giving it a bioresorption time ranging from 1 day to 3 months, preferably ranging from 1 to 8 weeks. The crosslinking of collagen by various crosslinking agents has been the subject of numerous studies and a person skilled in the art knows what degree of crosslinking to attribute to a collagen in order for the latter to have a rapid bioresorption according to the present application, that is to say ranging from 1 day to 3 months, for example from 1 to 8 weeks.

In one embodiment of the invention, use is made, as collagen with rapid *in vivo* bioresorption, of oxidized collagen, for example that is oxidized with periodic acid. Examples of the preparation of oxidized collagen suitable for the present invention are described in Patent US 6,596,304 by the Applicant.

It is also possible to vary the rate of degradation of the oxidized collagen, by modifying its degree of oxidation. It is known that the degree of oxidation of the collagen has an influence on the rate of bioresorption of the oxidized collagen. Thus, the lower the degree of oxidation of a collagen, the faster it will degrade and therefore be bioresorbed. As collagen having a low degree of oxidation, mention may be made of natural collagen, oxidized by periodic acid at a concentration of less than 10⁻² M, preferably between 10⁻⁴ M and 8 × 10⁻³ M, as described, for example, in French Patent FR 2,601,371. As collagen having a high degree of oxidation, mention may be made of natural collagen, oxidized by periodic acid at a concentration greater than 10⁻² M.

The collagen used for the collagen which undergoes rapid *in vivo* bioresorption may also be porcine collagen type I, extracted from porcine dermis by solubilization at acidic pH or by digestion with pepsin and purified by saline precipitations according to known techniques.

Use is preferably made of dry collagen fibres obtained by precipitation of an acid solution of collagen by addition of NaCl, then washing and drying of the precipitate obtained with aqueous solutions of acetone having a concentration increasing from 80% to 100%.

Alternatively, it is possible to use bovine or human collagens of types I, II, III, IV or V or a mixture thereof in any proportions.

In the case of human collagens of placental origin, they may be prepared by extraction with pepsin according to the method described in application EP-A 0 214 035.

The products sold by Inamed, under the names VITROGEN® or ZYDERM®, may also be suitable for the present invention.

In one embodiment of the invention, the collagen forming said sponge is a mixture of oxidized collagen and of collagen crosslinked with glutaraldehyde, the oxidized collagen having a degree of oxidation such that it is bioresorbed rapidly, and the crosslinked collagen having a degree of crosslinking that is sufficiently high for a slow bioresorption. The oxidized collagen may degrade *in vivo* and be bioresorbed over a few weeks. Conversely, said collagen crosslinked with glutaraldehyde is bioresorbed over several months. Thus, the degradation of the oxidized collagen creates, within the sponge matrix of the implant according to the invention, new pores that are interconnected with the pores of the knit and the cell growth may be distributed in a homogeneous, gradual and controlled manner, and may, little by little, invade the space left free by the degradation of the oxidized collagen. However, during the degradation of the oxidized collagen, the implant retains a sufficient mechanical strength due to the presence both of the collagen crosslinked with glutaraldehyde which itself degrades less quickly than the oxidized collagen, and of the three-dimensional knit.

The evolution of the interconnectivity of the implant after it has been implanted *in vivo* and the associated cell growth in the implant may be controlled by the respective nature and rate of degradation of the collagen which undergoes slow resorption and of the collagen which undergoes rapid resorption.

For example, if it is desired for the interconnectivity to increase rapidly within the final implant once it has been implanted *in vivo,* a collagen which undergoes rapid bioresorption that degrades particularly quickly will be provided, such as a not very oxidized collagen or natural collagen. The expression "natural collagen" is understood to mean a collagen that has not been chemically modified, other than by a possible treatment with pepsin that aims to remove the telomeres, therefore reducing its immunogenicity. The expression "not very oxidized collagen" is understood to mean a natural collagen, oxidized with periodic acid at a concentration of less than 10⁻² M, preferably between 10⁻⁴ M and 8 × 10⁻³ M, as described, for example, in French Patent FR 2,601,371.

Conversely, if a less rapid cell growth is desired, it will be possible to choose a collagen which undergoes rapid bioresorption that degrades less quickly such as a collagen having a high degree of oxidation or collagen crosslinked with crosslinking agents such as diglycidyl ethers, carbodiimides, acyl azides, divinyl sulphone, or glutaraldehyde at a low dose or collagen crosslinked by physical methods (UV, beta irradiation, gamma irradiation or photooxidation).

In order to obtain said sponge, a suspension comprising the oxidized collagen and the collagen crosslinked with glutaraldehyde is prepared. The suspension may comprise the two collagens in equal concentrations or conversely a majority of one of the two collagens and a minority of the other. The concentration ratio of one of the two types of collagen relative to the other is preferably between 1 and 5.

The evolution of the interconnectivity of the implant after it has been implanted *in vivo* and the associated cell growth in the implant may thus also be controlled by the ratio of concentrations between the collagen which undergoes slow resorption and the collagen which undergoes rapid resorption.

It is possible to vary the respective concentrations of the collagen which undergoes slow resorption and of the collagen which undergoes rapid resorption in the initial suspension, and therefore in the sponge obtained after freeze-drying according to the manner in which it is desired to change the degree of interconnectivity in the final implant and consequently the cell growth which is associated therewith. For example, if it is desired for the interconnectivity to increase rapidly within the final implant once it has been implanted *in vivo,* a predominant proportion of collagen which undergoes rapid resorption will be provided in the sponge of the matrix of the implant according to the invention. As this collagen which undergoes rapid resorption degrades, for example in a few days, it will be replaced with voids that will increase the interconnectivity of the implant and the cell growth will be able to take place rapidly within the numerous spaces left vacant by the degradation of the collagen which undergoes rapid resorption.

Conversely, if a less rapid cell growth is desired, a minority proportion of collagen which undergoes rapid resorption will be provided in the initial suspension for manufacturing the sponge of the matrix of the implant according to the invention.

In another embodiment of the invention, the collagen forming said sponge is a mixture of oxidized collagen and of collagen crosslinked with HMDI, the oxidized collagen having a degree of oxidation such that it is rapidly bioresorbed, and the crosslinked collagen having a degree of crosslinking that is sufficiently high for a slow bioresorption.

The implant according to the invention also comprises a porous three-dimensional knit having a longitudinal direction, said knit having at least one knit weave capable of giving it an elongation at 50 N in said longitudinal direction to strictly less than 10%, measured according to the ISO 13934-1 Standard.

According to the present application, the expression "three-dimensional knit" is understood to mean an assembly or an arrangement of biocompatible monofilament or multifilament yarns, obtained by knitting and it has a significant thickness. For example, the knit may have a thickness greater than or equal to 0. 5 mm.

In one embodiment of the invention, the three-dimensional knit comprises a first face and a second face, which are opposite one another and separated from one another by the thickness of the knit. The first and second faces are connected to one another by a spacer. For example, the spacer is composed of one or more lap(s) of connecting yarns. Each face may be composed of one or more laps of yarns. The yarns making up each of the two faces and the spacer may be identical or different.

One such embodiment of the knit of the implant according to the invention, with spacer yarns connecting a first face of the knit to a second face of the knit, helps to reinforce the interconnectivity of the pores, and in particular of the first pores, throughout the entire thickness of the collagen sponge, filling the three-dimensional knit. The interconnectivity of these pores may also be controlled, to a certain extent, by the density of the spacer yarns and their distribution between the two faces of the three-dimensional knit.

The knit of the implant according to the invention generally has an elongated shape, for example the shape of a rectangle: the longitudinal direction of the knit of the implant according to the invention corresponds to its largest dimension. In general, this direction is perpendicualr to the thickness of the knit.

In a manner known in the field of textiles, the knits have one or more knit weaves which constitute the knitting operating process or else the path or paths to follow for the yarns between the various needles of a knitting machine. For example, in a warp or Rashel machine, the yarns are threaded around guide bars which have predetermined movements in order to produce a particular knit.

The knit of the implant according to the invention has at least one knit weave which gives it an elongation at 50 N in said longitudinal direction that is strictly less than 10%, preferably ranging from 0.5% to 8%, meaesured according to the ISO 13934-1 Standard.

In one embodiment of the invention, the knit weave also gives the knit an elongation at 100 N in said longitudinal direction that is strictly less than 20%, preferably ranging from 1% to 15%, measured according to the ISO 13934-1 Standard.

In particular, the implant according to the invention is intended to replace and/or reinforce an orthopaedic soft tissue such as a ligament or a tendon. It is particularly preferred that, during the implantation of the implant, the longitudinal direction of the three-dimensional knit is aligned along the longitudinal direction or else the stress direction of the ligament or of the tendon to be treated.

Thus, the implant according to the invention is particularly effective for responding to the numerous stresses of the joint in which it is implanted by replacing or reinforcing the defective tendon or ligament. In particular, since the three-dimensional knit is not very elastic along its longitudinal direction, the implant correctly carries out its tendon or ligament role.

Thus, the knit of the implant of the invention comprises at least one weave of pillar stitch type positioned along its longitudinal direction. Pillar stitch type weaves are well known to a person skilled in the art in the textile field and further details will not be given here.

The three-dimensional knit of the implant of the invention comprises two opposite faces, a front face and a rear face, connected to one another by a spacer, at least one of said front and rear faces having at least one weave of pillar stitch type. More preferably, each of the two faces comprises at least one pillar stitch weave.

The weave of one face of the knit of the implant according to the invention may for example be defined by two laps of yarns, each of these two laps being obtained, for example, from a guide bar of a warp or Rashel machine, the chart followed for the knitting of the yarns of at least one of said laps resulting in a weave of pillar stitch type.

Such a configuration makes it possible to obtain a knit having both a thickness favourable to the regeneration of the tissue and a very low elasticity in the longitudinal direction of the knit and therefore, in particular, in the stress direction of the implant.

In one embodiment, the three-dimensional knit of the implant according to the invention has a warp strength, in other words strength in the longitudinal direction of the knit, ranging from 300 to 1000 N, measured according to the ISO 13934-1 Standard. In one embodiment, the three-dimensional knit of the implant according to the invention has a weft strength, in other words strength in the transverse direction of the knit, ranging from 300 to 1000 N, measured according to the ISO 13934-1 Standard.

In one embodiment of the implant of the invention, said three-dimensional knit of the implant according to the invention has a two-dimensional porosity of less than or equal to 20%.

For the purpose of the present application, the term "two-dimensional porosity" is intended to mean a porosity calculated from two-dimensional images corresponding to top views of the implant according to the invention, these images then being processed by software which analyses them, for instance the Image J software.

In one embodiment of the invention, the three-dimensional knit of the inplant according to the invention has a three-dimensional porosity of greater than or equal to 80%.

For the purpose of the present application, the term "three-dimensional porosity" is intended to mean a porosity measured in the following way: the dimensions, i.e. length, width and thickness, of the knit, taken alone, are measured; moreover, the density of the yarns used to knit this knit are known. The knit is weighed. By means of a simple subtraction, the volume occupied by the empty spaces within the knit is deduced therefrom. The three-dimensional porosity over the entire knit is determined as being the percentage of empty volume relative to the total volume of the knit.

Thus, preferably, the knit of the implant according to the invention has both a two-dimensional porosity of less than or equal to 20% and a three-dimensional porosity of greater than or equal to 80%. The applicant has noted, surprisingly, that the combination of these porosity values, which may appear to be paradoxical, makes it possible in particular to obtain, with the sponge forming the matrix of the implant according to the invention, an optimal interconnectivity for a better cell growth. Thus, when the implant according to the invention is manufactured, the collagen forming the sponge of the implant matrix has, by virtue of the high three-dimensional porosity of the knit of the implant according to the invention, a direct axis within the three-dimensional structure and therefore the pores of the knit. Moreover, the three-dimensional porosity of the knit of the implant according to the invention also makes it possible to limit as much as possible the mass of textile in the implant according to the invention, and therefore the mass of foreign body when it is implanted.

Furthermore, it is also advantageous for the knit of the implant according to the invention to have a relatively low two-dimensional porosity, preferably less than or equal to 20%, in order to maintain in the knit and therefore in the implant according to the invention, mechanical properties that are appropriate for the function that it is called upon to perform, i.e. reinforce a defective wall, in particular sufficient mechanical strength. The applicant has also noted that such a dimensional porosity of the three-dimensional knit contributes to forming interconnected pores in the collagen sponge, in all the dimensions of the sponge. Thus, the degree of interconnectivity of the pores of the collagen sponge, i.e. of the first pores, can also be controlled, to a certain extent, by the two-dimensional porosity of the three-dimensional knit, which can be made to vary between 0 and 20%.

The three-dimensional knit of the implant according to the invention is produced based on biocompatible monofilament or multifilament yarns. The three-dimensional knit of the implant according to the invention may comprise yarns made of a bioresorbable material, yarns made of a non-bioresorbable material or else a mixture of yarns made of bioresorbable material and of yarns made of non-bioresorbable material.

In one embodiment of the invention, said knit comprises at least one non-bioresorbable material and at least one bioresorbable material.

In one embodiment of the implant of the invention, said bioresorbable material is chosen from the group comprising polylactic acid (PLA), polyglycolic acid (PGA), oxidized cellulose, polycaprolactone (PCL), polydioxanone (PDO), trimethylene carbonate (TMC), polyvinyl alcohol (PVA), polyhydroxyalkanoates (PHAs), polyamides, polyethers, polysaccharides, copolymers thereof and mixtures thereof. The polysaccharides may be chosen from hyaluronic acid, alginic acid, polyglucuronic acid, chitosan, soluble derivatives of cellulose, the salts and the derivatives of these compounds, and mixtures thereof.

In one embodiment of the implant of the invention, said non-bioresorbable material is chosen from the group comprising polyesters, polyolefins, copolymers thereof and blends thereof. As a non-bioresorbable material, it is also possible to use non-bioresorbable cellulose.

For example, the yarns constituting the spacer may be made from a non-bioresorbable material. In one embodiment, the yarns constituting the opposite faces of the knit are a mixture of bioroesorbable yarns and of non-bioresorbable yarns.

In another embodiment of the invention, the knit of the implant of the invention is composed of a bioresorbable material, in other words said knit is completely bioresorbable.

Preferably, in one such embodiment, the bioresorbable material or materials constituting the yarns of the knit of the implant according to the invention have a resorption or degradation time *in vivo* that ranges from around 3 months to 2 years. The yarns constituting the knit may be in the form of a mixture of yarns having different bioresorption rates: thus, certain yarns are resorbed more quickly than others. Indeed, in the case where the implant is completely bioresorbable, it is preferable for the knit of the implant according to the invention to be the last component of the implant to be resorbed completely and to disappear in order to be replaced solely by regenerated tissue. Specifically, the knit of the implant according to the invention provides rigidity and some of the mechanical strength needed for the implant to play its reinforcing role.

For example, as yarns having a rapid bioresorption rate, mention may be made of yarns made of oxidized cellulose, these yarns may be obtained according to any method known to a person skilled in the art for oxidizing cellulose. Preferably, the oxidized cellulose is chosen from the oxidized celluloses in which the primary alcohol at C₆ is partially or completely oxidized to carboxylic acid, for example to give polyglucuronic acid, the cellulose oxidized by periodic acid to give polyaldehydes, the "viscous"-type cellulose obtained from a paste of cellulose that has been dissolved and then regenerated and oxidized, and mixtures thereof.

In one embodiment of the invention, the first and second faces of the knit are identical. For example, each face is composed of two laps of yarns.

Preferably, the yarns constituting the two faces of the knit are made of multifilament yarns of polylactic acid. Such yarns are completely resorbed *in vivo* in the space of 6 months to 2 years.

In one embodiment of the invention, the yarns constituting the spacer are monofilament yarns. Such an embodiment makes it possible to give the knit a better mechanical strength and a better resistance to thermosetting in the case where the knit is thermoset after the knitting phase. Preferably, the spacer is produced from monofilament yarns of polylactic acid.

In one embodiment, at least one portion of the yarns constituting the three-dimensional knit are covered with a bioresorbable coating. The bioresorbable coating may be chosen from oxidized collagen, collagen crosslinked with glutaraldehyde, biofunctional or trifunctional glycidyl ethers, carbodiimides, acyl azides, divinylsulphone, collagen crosslinked by UV, beta or gamma irradiation or by heat treatment, and mixtures thereof. All of the yarns constituting said knit may be covered with such a coating. For example, the coating is made of collagen. In particular, for such a coating it is possible to use a collagen chosen from the group comprising oxidized collagen, collagen crosslinked with glutaraldehyde and mixtures thereof.

In one embodiment, the yarns of the knit are covered, at least partly, by coating the knit in a solution or suspension of collagen, in one step or in several steps. A coating step comprises the actual coating of the knit by the collagen and the drying of the knit. The collagen deposited on the yarns may be crosslinked by the glutaraldehyde after each application as many times as the total number of coating cycles. Preferably, the yarns are covered by carrying out two to three successive coating cycles.

In another embodiment, the bioresorbable coating may be chosen from polysaccharides including hyaluronic acid, alginic acid, polyglucuronic acid, chitosan, starch, soluble cellulose derivatives and mixtures thereof.

In another embodiment, before being coated with the bioresorbable coating described above, the knit according to the invention may be subjected to a surface treatment in order to make it more hydrophilic and thus promote the deposition of the collagen and/or of the polysaccharides mentioned above on the knit.

The surface treatment may be carried out according to any process known to a person skilled in the art.

Such a coating of the yarns makes it possible, in particular, to eliminate any possible crevice within the knit of the implant according to the invention, for example where the yarns cross, crevices liable to create sites where bacteria or inflammatory cells develop.

Such an implant thus makes it possible to reduce the risks of inflammation and of sepsis, the bioresorbable coating making the accessible surface of the knit completely smooth and thus preventing the installation of undesirable bacteria and/or microorganisms and/or inflammatory cells.

In order to produce the implant according to the invention, the knit as described above is first knitted on a knitting machine. A three-dimensional knit may be obtained on a warp or Rashel type rib knitting machine using, for example, 5 or 6 guide bars. Preferably this knit is thermoset, for example by being placed in an oven at from 100 to 250°C, for 30 s to 5 minutes, depending on the chemical nature of the yarns used. The knit is then cut to the dimensions desired for the implant, preferably according to an elongated shape, remembering to have the longitudinal direction of the knit in the direction intended to be the stress direction of the implant, that is to say the main direction in which it will be stressed. The thermosetting may also be carried out after the knit has been cut up.

A suspension comprising the collagen intended to form the sponge of the matrix is then prepared. For example, this suspension comprises a mixture of collagen which undergoes rapid resorption and collagen which undergoes slow resorption. The collagen suspension is then poured over the three-dimensional knit so as to completely cover it. The whole is then freeze-dried, for example according to the following method: freezing is carried out as rapidly as possible, by decreasing the temperature of the product from 8°C to - 45°C, generally in less than 2 hours. Primary desiccation is initiated at -45°C, at a pressure of from 0.1 to 0.5 mbar. During this step, the temperature is gradually increased, with successive slopes and plateaux, to +30°C. The freeze-drying generally ends with secondary desiccation, at +30°C, for 1 to 24 hours. The vacuum at the end of secondary desiccation is preferably between 0.005 and 0.2 mbar. The total freeze-drying time is from 18 to 72 hours.

The freeze-drying makes it possible to obtain an implant in which all the pores, i.e. the first pores, formed with the sponge, and the second pores, i.e. those of the three-dimensional knit present prior to the freeze-drying, are at least partially interconnected.

The implant according to the invention can also be coated, on at least one of its faces, with a bioresorbable film. This film is preferably smooth at the surface and can be used for the prevention of post-surgical adhesions.

Such a film may be a collagen film. In one embodiment of the invention, such a film comprises oxidized collagen, polyethylene glycol and glycerol.

This bioresorbable film can be applied to one face of the implant according to the invention in the following way: a solution, for example of oxidized collagen, polyethylene glycol and glycerol, is prepared and then spread out in order to form a thin layer on a hydrophobic flat support, for example on a support of polyvinyl chloride or polystyrene. The face of the implant to be coated can then be applied carefully to the collagen gel. After exposure to ambient temperature and evaporation, a film which coats one face of the implant is obtained. It is also possible to coat the two faces of the implant with such a film, so that the matrix of the implant is sandwiched between the two bioresorbable films. This film preferably is resorbed rapidly *in vivo,* for example in less than 8 days.

The implant according to the invention may also comprise one or more active compounds for improving the repair of walls and tissues, especially chosen from antiseptic agents, anti-inflammatory agents, growth factors, polysaccharides such as fucans, extracellular matrix proteins such as fibronectin, laminin or elastin, glycosaminoglycans, proteoglycans, and mixtures thereof.

The following examples illustrate the invention.

### EXAMPLES:

### EXAMPLE 1: Preparation of a knit for the implant according to the invention

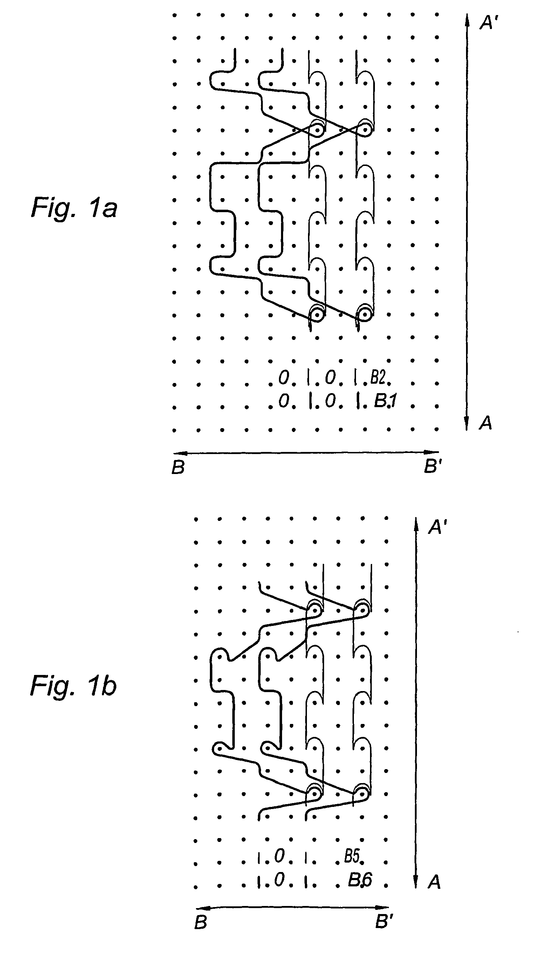

A three-dimensional knit is produced on a double needle bar Rashel knitting machine, with 5 guide bars B1, B2, B3, B5 and B6. Each of the faces of the knit, namely the first face (or front face) and the second face (or rear face), is produced with two guide bars, B1 and B2 for the first face, B5 and B6 for the second face. With reference to Figure 1a, the bar B1 is threaded by the yarns represented by bold lines and the bar B2 is threaded by the yarns represented as fine lines. With reference to Figure 1b, the bar B5 is threaded by yarns represented by fine lines and the bar B6 is threaded by yarns represented by bold lines. The first face is produced with the bars B1 and B2, and the second opposite face is produced with the bars B5 and B6, each bar being threaded one full, one empty, with the following respective charts, corresponding to a customary system for describing knit weaves that is comprehensible to a person skilled in the art:
Bar B1: 1-0-3-3/5-5-4-4/4-4-5-5/5-5-3-3//.
Bar B2: 1-0-0-0/0-1-1-1//.
Bar B5: 1-1-1-0/0-0-0-1//.
Bar B6: 3-3-0-1/3-3-5-4/4-4-4-4/5-5-5-4//.

The weave corresponding to the bars B1 and B2 is reproduced in Figure 1a. The weave corresponding to the bars B5 and B6 is reproduced in Figure 1 b. Such weaves result in porous faces. Furthermore, the bar B2 and the bar B5 each produce a pillar stitch weave.

The bars B1-B2 and B5-B6 producing the first and second faces of the knit are threaded with multifilament PET (polyethylene terephthalate) yarns, 1 full, 1 empty.

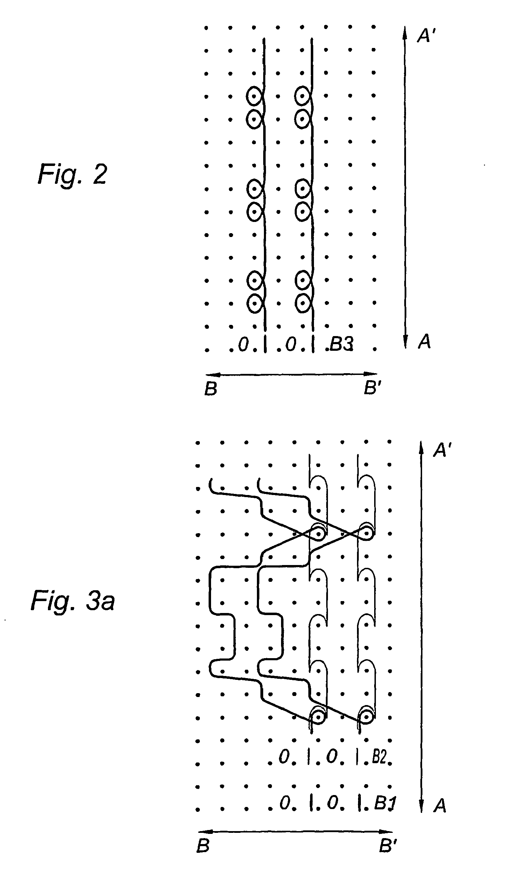

With reference to Figure 2, the spacer is produced using the bar B3 threaded with multifilament PET yarns, 1 full, 1 empty, according to the following chart:
Bar B3: 0-1-0-1/0000//.

The weave of bar B3 results in a porous spacer.

In Figures 1a, 1b and 2, the arrow AA' indicates the longitudinal direction of the knit, also called the warp direction for the measurements of the mechanical properties in the present example, and the arrow BB' indicates the transverse direction of the knit, also called the weft direction for the measurements of the mechanical properties in the present example.

The knit is de-sized with methanol-ether.

The knit is then thermoset by placing it in an oven at around 200°C for 1 to 5 min.

Such a knit has the following properties, measured as indicated in the present application:
- Three-dimensional porosity: 92%
- Two-dimensional porosity: 18%

The knit according to the present example also has the following mechanical properties:

| Property | Knit EXAMPLE 1 |
|---|---|
| Wa Str in N ± standard deviation | 611 ± 19 |
| We Str in N ± standard deviation | 483 ± 58 |
| M Wa El in % ± standard deviation | 39.6 ± 2.5 |
| M We El in % ± standard deviation | 64.4 ± 6.6 |
| 50 N Wa El in % ± standard deviation | 1.9 ± 0.04 |
| 50 N We El in % ± standard deviation | 11.7 ± 0.5 |
| 100 N Wa El in % ± standard deviation | 3.8 ± 0.08 |
| 100 N We El in % ± standard deviation | 22.8 ± 0.3 |

| | |
|---|---|
| Wa Str: Warp strength (in N); We Str: Weft strength (in N); calculated according to the ISO 13934-1 Standard M Wa El: Maximum warp elongation (in %) calculated according to the ISO 13934-1 Standard; M We El: Maximum weft elongation (in %) calculated according to the ISO 13934-1 Standard; 50 N Wa El: Warp elongation at 50 N (in %) calculated according to the ISO 13934-1 Standard; 50 N We El: Weft elongation at 50 N (in %) calculated according to the ISO 13934-1 Standard; 100 N Wa El: Warp elongation at 50 N (in %) calculated according to the ISO 13934-1 Standard; 100 N We El: Weft elongation at 50 N (in %) calculated according to the ISO 13934-1 Standard | |

Thus, the knit of the present example according to the invention has a warp elongation, that is to say an elongation in its longitudinal direction, at 50 N of around 1.9% and at 100 N of around 3.8%. Such a knit is particularly not very elastic in its longitudinal direction.

### EXAMPLE 2: Preparation of a knit for the implant according to the invention

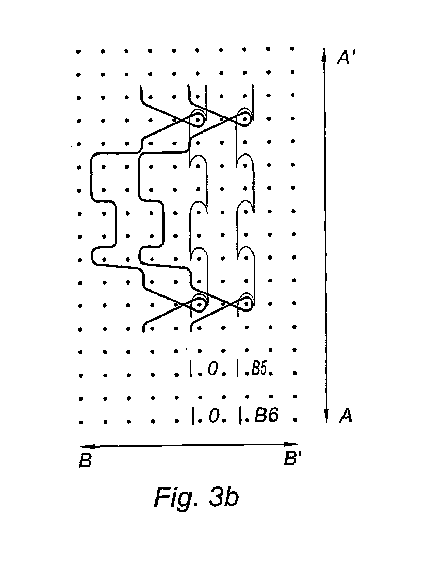

A three-dimensional knit is produced on a double needle bar Rashel knitting machine, with 5 guide bars B1, B2, B3, B5 and B6. Each of the faces of the knit, namely the first face (or front face) and the second face (or rear face), is produced with two guide bars, B1 and B2 for the first face, B5 and B6 for the second face. With reference to Figure 3a, the bar B1 is threaded by the yarns represented by bold lines and the bar B2 is threaded by the yarns represented as fine lines. With reference to Figure 3b, the bar B5 is threaded by yarns represented by fine lines and the bar B6 is threaded by yarns represented by bold lines. The first face is produced with the bars B1 and B2, and the second opposite face is produced with the bars B5 and B6, each bar being threaded one full, one empty, with the following respective charts, corresponding to a customary system for describing knit weaves that is comprehensible to a person skilled in the art:
Bar B1: 1-0-3-3/5-5-4-4/4-4-5-5/5-5-3-3//.
Bar B2: 1-0-0-0/0-1-1-1//.
Bar B5: 1-1-1-0/0-0-0-11//.
Bar B6: 3-3-1-0/3-3-5-5/4-4-4-4/5-5-5-5//.

The weave corresponding to the bars B1 and B2 is reproduced in Figure 3a. The weave corresponding to the bars B5 and B6 is reproduced in Figure 3b. Such weaves result in porous faces. Furthermore, the bar B2 and the bar B5 each produce a pillar stitch weave.

The bars B1-B2 and B5-B6 producing the first and second faces of the knit are threaded with multifilament PET yarns, 1 full, 1 empty.

With reference to Figure 2, the spacer is produced using the bar B3, as in EXAMPLE 1 above, threaded with multifilament PET yarns, 1 full, 1 empty, according to the following chart:
Bar B3: 0-1-0-1/0-0-0-0//.

The weave of bar B3 results in a porous spacer.

In Figures 2, 3a and 3b, the arrow AA' indicates the longitudinal direction of the knit, also called the warp direction for the measurements of the mechanical properties in the present example, and the arrow BB' indicates the transverse direction of the knit, also called the weft direction for the measurements of the mechanical properties in the present example.

The knit is de-sized with methanol-ether.

The knit is then thermoset by placing it in an oven at around 200°C for 1 to 5 min.

Such a knit has the following properties, measured as indicated in the present application:
- Three-dimensional porosity: 83%
- Two-dimensional porosity: 7%

This knit has the following mechanical properties:

| Property | Knit EXAMPLE 2 |
|---|---|
| Wa Str in N ± standard deviation | 526 ± 14 |
| We Str in N ± standard deviation | 563 ± 29 |
| M Wa El in % ± standard deviation | 52.1 ± 1.9 |
| M We El in % ± standard deviation | 33.1 ± 2.4 |
| 50 N Wa El in % ± standard deviation | 6.3 ± 0.7 |
| 50 N We El in % ± standard deviation | 8.5 ± 0.6 |
| 100 N Wa El in % ± standard deviation | 12.6 ± 0.8 |
| 100 N We El in % ± standard deviation | 13.4 ± 0.6 |

| | |
|---|---|
| Wa Str: Warp strength (in N); We Str: Weft strength (in N); calculated according to the ISO 13934-1 Standard M Wa El: Maximum warp elongation (in %) calculated according to the ISO 13934-1 Standard; M We El: Maximum weft elongation (in %) calculated according to the ISO 13934-1 Standard; 50 N Wa El: Warp elongation at 50 N (in %) calculated according to the ISO 13934-1 Standard; 50 N We El: Weft elongation at 50 N (in %) calculated according to the ISO 13934-1 Standard; 100 N Wa El: Warp elongation at 50 N (in %) calculated according to the ISO 13934-1 Standard; 100 N We El: Weft elongation at 50 N (in %) calculated according to the ISO 13934-1 Standard | |

Thus, the knit of the present example according to the invention has a warp elongation, that is to say an elongation in its longitudinal direction, at 50 N, of around 6.3% and at 100 N of around 12.6%. Such a knit is particularly not very elastic in its longitudinal direction.

### EXAMPLE 3:

### 1°) Coating of a knit according to the invention

A knit according to the invention of the type of that obtained in EXAMPLE 1 could be coated with a 0.8% w/v porcine collagen solution, according to the following procedure.

The knit is dipped in the solution, it is wrung out and it is left to dry under a laminar flow. This cycle of operations is repeated up to two times in order to obtain covering of the yarns.

The collagen used is porcine collagen type I, extracted from porcine dermis by solublization at acidic pH or by digestion with pepsin and purified by saline precipitations according to known techniques.

Use is preferably made of dry collagen fibres obtained by precipitation of an acid solution of collagen by addition of NaCl, then washing and drying of the precipitate obtained with aqueous solutions of acetone having a concentration increasing from 80% to 100%.

At the end of the coating process, the collagen deposited on the knit is crosslinked with glutaraldehyde at 0.5% w/v (25% w/v aqueous solution of glutaraldehyde sold by Fluka), at neutral pH (pH between 6.5 and 7.5) for 2 hours, and is then reduced with sodium borohydride. The reactants used are removed by washing the knit using several waterbaths.

The crosslinking of the collagen deposited on the knit can alternatively be carried out at the end of each coating cycle.

### 2°) Preparation of an implant according to the invention:

An implant according to the invention could be prepared according to the procedures described below:

### a) Preparation of collagen crosslinked with glutaraldehyde

Porcine collagen is solubilized in water at a final concentration of 1% w/v.

The collagen used is porcine collagen type I, extracted from porcine dermis by solubilization at acidic pH or by digestion with pepsin, and purified by saline precipitations according to known techniques.

Dry collagen fibres obtained by precipitation of an acid solution of collagen by adding NaCl, and then washing and drying the precipitate obtained with aqueous solutions of acetone having an concentration increasing from 80% to 100%, are preferably used.

The solution of collagen at 1% w/v is then neutralized by adding sodium phosphate at a final concentration of 20mM. The final pH of the suspension is between 6.5 and 7.5.

Glutaraldehyde (25% w/v aqueous solution of glutaraldehyde, sold by Fluka) is then added to the suspension at a final concentration of 0.5% w/v. After two hours at ambient temperature, collagen fibres are recovered by filtration of the suspension through a nylon mesh. These fibres are then treated with sodium borohydride for at least two hours until the yellow coloration of the fibres has completely disappeared. The white fibres thus obtained are washed and neutralized at pH 6.5-7.5, and dried by removing the water with acetone. The acetone residues are then evaporated off.

### b) Preparation of oxidized collagen

A 3% w/v porcine collagen solution is oxidized with periodic acid at a final concentration of 8 mM, at ambient temperature, according to Example 4 of US 6,596,304.

### c) Preparation of the implant:

A suspension of collagen is prepared by mixing the collagen crosslinked with glutaraldehyde and the oxidized collagen obtained in points a) and b) above, in the following concentrations:
- 0.5 to 1.5% w/v of collagen crosslinked with glutaraldehyde,
- 0.2 to 1 % w/v of oxidized collagen.

The collagen suspension thus obtained is then poured over the three-dimensional knit from point 1) above so as to completely cover it and the whole is freeze-dried according to the following method: freezing is carried out as rapidly as possible, by decreasing the temperature of the product from 8°C to -45°C, generally in less than 2 hours. Primary desiccation is initiated at - 45°C, at a pressure of from 0.1 to 0.5 mbar. During this step, the temperature is gradually increased, with successive slopes and plateaux, to +30°C. The freeze-drying ends with secondary desiccation, at +30°C, for 1 to 24 hours. Preferably, the vacuum at the end of secondary desiccation is between 0.005 and 0.2 mbar. The total freeze-drying time is from 18 to 72 hours.

An implant in which all the pores, i.e. those formed with the sponge and those of the three-dimensional knit, are at least partially interconnected, can thus be obtained.

### 3°) Application of a film to one face of the implant:

A film could also be applied to one face of the implant obtained in point 2°) above by covering it with an oxidized collagen film as described in Example 2 of US 6, 391, 939.

The procedure that can be used is the following:
A concentrated sterile solution of PEG 4000 (polyethylene glycol having a molecular weight of 4000 D, for example sold by Fluka under the trade name PEG 4000) and glycerol is added to a 3% w/v solution of oxidized collagen (obtained by oxidation of porcine collagen), so as to obtain a final composition having a PEG 4000 concentration of 1% w/v and a glycerol concentration of 0.6% w/v. The pH of the solution is adjusted to 7.0 by adding a concentrated solution of sodium hydroxide. The volume of the solution is then adjusted with sterile water so as to obtain final concentrations of collagen, of PEG 4000 and of glycerol of 2.7% w/v, 0.9% w/v and 0.54% w/v, respectively. The solution is then spread out so as to form a thin layer with a density of 0.133 g/cm² on a flat hydrophobic support of polyvinyl chloride or polystyrene type. The surface is then exposed to a stream of sterile air at ambient temperature for just under one hour.

The implant obtained in point 2°) above is then applied carefully to the gelled layer of oxidized collagen above. The whole is exposed to a stream of sterile air at ambient temperature until complete evaporation in about 18 hours.

An implant particularly suitable for repairing orthopaedic soft tissues can thus be obtained.

### EXAMPLE 4:

An implant according to the invention could also be prepared from a three-dimensional knit suitable for the present invention coated with a coating of collagen crosslinked with glutaraldehyde of the type of that described in EXAMPLE 3, 1°) above, using the following procedures:

### 1°) Preparation of the implant:

### a) Preparation of collagen crosslinked with HMDI:

50 g of dry porcine collagen is mixed with 1 l of acetone. 1 g of HMDI is then added to the collagen suspension. The mixture is left overnight, with stirring and at ambient temperature (around 20°C). The collagen fibres are then recovered by filtering the suspension through a nylon mesh and are carefully washed with dry acetone in order to remove the HMDI and the by-products of HMDI that are soluble in acetone. The crosslinked collagen fibres thus obtained are dried by removing the residues of acetone. The fibres may then be optionally ground.

### b) Preparation of oxidized collagen:

The oxidized collagen is prepared as in EXAMPLE 3, 2°)b) above.

### c) Preparation of the implant:

A suspension of collagen is prepared by mixing the collagen crosslinked with HMDI and the oxidized collagen obtained in points a) and b) above in the following concentrations:
- 0.5 to 1.5% w/v of collagen crosslinked with HMDI;
- 0.2 to 1 % w/v of oxidized collagen.

The collagen suspension thus obtained is then poured over the three-dimensional knit above so as to completely cover it and the whole is freeze-dried as described in EXAMPLE 3.

### 2°) Application of a film to each face of the implant:

A first face of the implant obtained in point 2°) above is covered with a film of oxidized collagen as described in EXAMPLE 3 above.

Then a second film of oxidized collagen is applied to the other face of the implant: a solution of oxidized collagen is spread out so as to form a thin layer with a density of 0.133 g/cm² on a flat hydrophobic support of polyvinyl chloride or polystyrene type. The second (uncoated) face of the implant is applied gently to the gelled layer of oxidized collagen. The whole is then exposed to a stream of sterile air at ambient temperature for just under an hour.

An implant is thus obtained that is capable, in an infected implantation site, of arresting the infectious phenomenon, while the two films degrade and are bioresorbed, until healthy cells are present.

### 3°) Additional crosslinking of the implant:

The implant obtained in point 2°) above may be treated with a 0.1 % w/v solution of HMDI in isopropanol. The implant is treated in such a solution for 20 hours. It is then washed several times with isopropanol. The solvent is removed by evaporation.

Such a treatment results in a crosslinking of the whole of the implant. It is thus possible, if desired, to increase the time needed for the bioresorption of the whole of the implant.

### EXAMPLE 5:

It would also be possible to coat the knits from EXAMPLES 1 and 2 with chitosan in a step according to the following procedure: each knit is coated with a 1% chitosan solution (degree of acetylation: 2.5%; high molecular weight chitosan, chitosan extract, Mahtani Chitosan Pvt Ltd), by spraying said chitosan solution onto the knit, until the knit is completely wetted. Each knit is then dried at +50°C. This cycle of operations is repeated up to four times to obtain the covering of the yarns.

## Claims

1. Implant intended to replace or reinforce defective orthopaedic soft tissue, such as a tendon or ligament, comprising:
- a bioresorbable porous matrix based on a collagen sponge;
- a porous three-dimensional knit having a longitudinal direction, said porous matrix filling said three-dimensional knit,
**characterized in that**:
said three-dimensional knit having two opposite faces, a front face and a rear face, connected to one another by a spacer, at least one of said front and rear faces has at least one weave of pillar stitch type in the longitudinal direction of said knit.

2. Implant according to Claim 1, **characterized in that** each of the front and rear faces of said three-dimensional knit has at least one weave of pillar stitch type.

3. Implant according to one of Claims 1 or 2, **characterized in that** said collagen is a mixture of at least one collagen which undergoes slow *in vivo* bioresorption and at least one collagen which undergoes rapid *in vivo* bioresorption.

4. Implant according to Claim 3, **characterized in that** the collagen which undergoes slow *in vivo* bioresorption is a collagen crosslinked with a crosslinking agent chosen from glutaraldehyde, hexamethylene diisocyanate (HMDI), bifunctional or trifunctional glycidyl ethers, carbodiimides, acyl azides, divinyl sulphone and mixtures thereof, the degree of crosslinking of said crosslinked collagen giving it a bioresorption time ranging from 3 months to 12 months.

5. Implant according to Claim 4, **characterized in that** said collagen which undergoes slow *in vivo* bioresorption is a collagen crosslinked with glutaraldehyde.

6. Implant according to Claim 4, **characterized in that** said collagen which undergoes slow *in vivo* bioresorption is a collagen crosslinked with hexamethylene diisocyanate (HMDI).

7. Implant according to one of Claims 3 to 6, **characterized in that** the collagen which undergoes rapid *in vivo* bioresorption is an oxidized collagen or a collagen crosslinked with a crosslinking agent chosen from glutaraldehyde, bifunctional or trifunctional glycidyl ethers, carbodiimides, acyl azides, divinyl sulphone and mixtures thereof, the degree of crosslinking of said crosslinked collagen giving it a bioresorption time ranging from 1 day to 3 months, preferably ranging from 1 to 8 weeks.

8. Implant according to Claim 7, **characterized in that** the collagen which undergoes rapid *in vivo* bioresorption is an oxidized collagen.

9. Implant according to one of Claims 1 to 8, **characterized in that**, said matrix defining first pores, and said knit defining second pores, said first and second pores are at least partially interconnected with one another.

10. Implant according to one of Claims 1 to 9, **characterized in that** said knit comprises at least one non-bioresorbable material and at least one bioresorbable material.

11. Implant according to Claim 10, **characterized in that** the knit comprises at least one 50%, by weight, relative to the total weight of the knit, of bioresorbable material.

12. Implant according to one of Claims 1 to 9, **characterized in that** said knit is composed of bioresorbable material.

13. Implant according to one of Claims 1 to 12, **characterized in that** it also comprises a bioresorbable film on at least one of its faces.

14. Implant according to Claim 13, **characterized in that** it comprises a bioresorbable film on each of its two faces, so that said matrix is sandwiched between the two bioresorbable faces.

## Patentansprüche

1. Implantat, das vorgesehen ist, um defektes orthopädisches Weichgewebe wie eine Sehne oder ein Band zu ersetzen oder zu verstärken, umfassend:
- eine bioresorbierbare poröse Matrix, die auf einem Kollagenschwamm basiert;
- ein poröses dreidimensionales Gestrick mit einer Längsrichtung, wobei die poröse Matrix das dreidimensionale Gestrick füllt,
**dadurch gekennzeichnet, dass**:
das dreidimensionale Gestrick zwei gegenüberliegende Flächen, eine Vorderfläche und eine Rückfläche, aufweist, die über einen Abstandhalter miteinander verbunden sind, wobei zumindest eine der Vorder- und Rückflächen zumindest ein Gewebe vom Kettenstichtyp in der Längsrichtung des Gestricks aufweist.

2. Implantat nach Anspruch 1, **dadurch gekennzeichnet, dass** jede der Vorder- und Rückflächen des dreidimensionalen Gestricks zumindest ein Gewebe vom Kettenstichtyp aufweist.

3. Implantat nach einem der Ansprüche 1 oder 2, **dadurch gekennzeichnet, dass** das Kollagen ein Gemisch aus zumindest einem Kollagen, das *in vivo* langsam bioresorbiert wird, und zumindest einem Kollagen, das *in vivo* schnell bioresorbiert wird, ist.

4. Implantat nach Anspruch 3, **dadurch gekennzeichnet, dass** das Kollagen, das *in vivo* langsam bioresorbiert wird, ein Kollagen ist, das mit einem Vernetzungsmittel vernetzt ist, das aus Glutaraldehyd, Hexamethylendiisocyanat (HMDI), bifunktionellen oder trifunktionellen Glycidylestern, Carbodiimiden, Acylaziden, Divinylsulphon und Mischungen davon ausgewählt ist, wobei der Grad der Vernetzung des vernetzten Kollagens dem Kollagen eine Bioresorptionszeit im Bereich von 3 Monaten bis 12 Monaten verleiht.

5. Implantat nach Anspruch 4, **dadurch gekennzeichnet, dass** das Kollagen, das *in vivo* langsam bioresorbiert wird, ein mit Glutaraldehyd vernetztes Kollagen ist.

6. Implantat nach Anspruch 4, **dadurch gekennzeichnet, dass** das Kollagen, das *in vivo* langsam bioresorbiert wird, ein mit Hexamethylendiisocyanat (HMDI) vernetztes Kollagen ist.

7. Implantat nach einem der Ansprüche 3 bis 6, **dadurch gekennzeichnet, dass** das Kollagen, das *in vivo* schnell bioresorbiert wird, ein oxidiertes Kollagen oder ein mit einem Vernetzungsmittel vernetztes Kollagen ist, wobei das Vernetzungsmittel aus Glutaraldehyd, bifunktionellen oder trifunktionellen Glycidylestern, Carbodiimiden, Acylaziden, Divinylsulphon und Mischungen davon ausgewählt ist, wobei der Grad der Vernetzung des vernetzten Kollagens dem Kollagen eine Bioresorptionszeit im Bereich von 1 Tag bis 3 Monaten, vorzugsweise im Bereich von 1 bis 8 Wochen, verleiht.

8. Implantat nach Anspruch 7, **dadurch gekennzeichnet, dass** das Kollagen, das *in vivo* schnell bioresorbiert wird, ein oxidiertes Kollagen ist.

9. Implantat nach einem der Ansprüche 1 bis 8, **dadurch gekennzeichnet, dass** die Matrix erste Poren definiert, und dass das Gestrick zweite Poren definiert, wobei die ersten und zweiten Poren zumindest teilweise miteinander verbunden sind.

10. Implantat nach einem der Ansprüche 1 bis 9, **dadurch gekennzeichnet, dass** das Gestrick zumindest ein nicht-bioresorbierbares Material und zumindest ein bioresorbierbares Material umfasst.

11. Implantat nach Anspruch 10, **dadurch gekennzeichnet, dass** das Gestrick zumindest 50 Gew.-% bioresorbierbares Material in Bezug auf das Gesamtgewichts des Gestricks umfasst.

12. Implantat nach einem der Ansprüche 1 bis 9, **dadurch gekennzeichnet, dass** das Gestrick aus bioresorbierbarem Material hergestellt ist.

13. Implantat nach einem der Ansprüche 1 bis 12, **dadurch gekennzeichnet, dass** es darüber hinaus auf zumindest einer seiner Flächen einen bioresorbierbaren Film umfasst.

14. Implantat nach Anspruch 13, **dadurch gekennzeichnet, dass** es einen bioresorbierbaren Film auf jede seiner zwei Flächen umfasst, so dass die Matrix zwischen den beiden bioresorbierbaren Flächen eingeklemmt ist.

## Revendications

1. Implant destiné à remplacer ou renforcer un tissu mou orthopédique défectueux, tel qu'un tendon ou ligament, comprenant :
- une matrice poreuse biorésorbable à base d'une éponge de collagène,
- un tricot tridimensionnel poreux présentant une direction longitudinale, ladite matrice poreuse remplissant ledit tricot tridimensionnel, **caractérisé en ce que** :
- ledit tricot tridimensionnel comporte deux faces opposées, une face avant et une face arrière, reliées entre elles par une entretoise, au moins une desdites faces avant et arrière présente au moins une armure de type chaînette, selon la direction longitudinale dudit tricot.

2. Implant selon la revendication 1, **caractérisé en ce que** chacune des faces avant et arrière dudit tricot tridimensionnel présente au moins une armure de type chaînette.

3. Implant selon l'une des revendications 1 ou 2, **caractérisé en ce que** ledit collagène est un mélange d'au moins un collagène à biorésorption in vivo lente et au moins un collagène à biorésorption in vivo rapide.

4. Implant selon la revendication 3, caractérisé en que le collagène à biorésorption in vivo lente est un collagène réticulé avec un agent de réticulation choisi parmi le glutaraldéhyde, l'hexaméthylènediisocyanate (HMDI), les glycidyl éthers bi- ou tri-fonctionnels, les carbodiimides, les acyl azides, la divinylsulfone et leurs mélanges, le degré de réticulation dudit collagène réticulé lui conférant un temps de biorésorption allant de 3 mois à 12 mois.

5. Implant selon la revendication 4, **caractérisé en ce que** ledit collagène à biorésorption in vivo lente est un collagène réticulé avec du glutaraldéhyde.

6. Implant selon la revendication 4, **caractérisé en ce que** ledit collagène à biorésorption in vivo lente est un collagène réticulé avec de l'hexaméthylènediisocyanate (HMDI).

7. Implant selon l'une des revendication 3 à 6, **caractérisé en ce que** le collagène à biorésorption in vivo rapide est un collagène oxydé ou un collagène réticulé avec un agent de réticulation choisi parmi le glutaraldéhyde, les glycidyl éthers bi- ou tri-fonctionnels, les carbodiimides, les acyl azides, la divinylsulfone et leurs mélanges, le degré de réticulation dudit collagène réticulé lui conférant un temps de biorésorption allant de 1 jour à 3 mois, de préférence allant de 1 à 8 semaines.

8. Implant selon la revendication 7, **caractérisé en ce que** ledit collagène à biorésorption in vivo rapide est un collagène oxydé.

9. Implant selon l'une des revendications 1 à 8, **caractérisé en ce que**, ladite matrice définissant des premiers pores, et ledit tricot définissant des deuxièmes pores, lesdits premiers et deuxièmes pores sont au moins partiellement interconnectés les uns avec les autres.

10. Implant selon l'une des revendications 1 à 9, **caractérisé en ce que** ledit tricot comprend au moins un matériau non biorésorbable et au moins un matériau biorésorbable.

11. Implant selon la revendication 10, **caractérisé en ce que** le tricot comprend au moins 50%, en poids, par rapport au poids total du tricot, de matériau biorésorbable.

12. Implant selon l'une des revendications 1 à 9, **caractérisé en ce que** ledit tricot est constitué de matériau biorésorbable.

13. Implant selon l'une des revendications 1 à 12, **caractérisé en ce qu'**il comprend en outre un film biorésorbable sur au moins l'une de ses faces.

14. Implant selon la revendication 13, **caractérisé en ce que** qu'il comprend un film biorésorbable sur chacune de ses deux faces, de telle sorte que ladite matrice est prise en sandwich entre les deux films biorésorbables.
